# EUROPEAN PATENT APPLICATION

(11) **EP 2 022 507 A1**
(43) Date of publication of application: **11.02.2009**
(21) Application number: 07015510.6
(22) Date of filing: 07.08.2007
(51) Int. Cl.: A61K 39/35, C07K 16/16, C07K 16/18

(54) **Antibody compositions specific for lgE, lgG4 and lgA epitopes as tools for the design of hypoallergenic molecules for specific immunotherapy**

(71) Applicant: Universität Hamburg, 20146 Hamburg (DE)
(72) Inventor: Braren, Ingke, 22303 Hamburg (DE); Bredehorst, Reinhard Prof., 20255 Hamburg (DE); Ollert, Markus Prof. Dr., 82131 Gauting (DE); Grunwald, Thomas Dr., 22459 Hamburg (DE); Ring, Johannes Prof. Dr., 82057 Icking (DE); Spillner, Edzard Dr., 22765 Hamburg (DE)
(74) Representative: UEXKÜLL & STOLBERG

(57) **Abstract**

The invention relates to a composition comprising recombinant antibodies capable of binding to epitopes recognized by antibodies of a specific isotype, in particular, human IgE, IgG4 and/or IgA antibodies, a method of obtaining such a composition and the use of individual antibodies of such a composition as tools for the design of hypoallergenic molecules for specific immunotherapy, as well as a method for generating such hypoallergenic molecules.

## Description

The present invention relates to a composition comprising recombinant antibodies capable of binding to epitopes recognized by human IgE, IgG4 and IgA antibodies, a method of obtaining such a composition and the use of individual antibodies of such a composition as tools for the design of hypoallergenic molecules for specific immunotherapy, as well as a method for producing hypoallergenic molecules for specific immunotherapy.

### BACKGROUND

More than 20% of the population suffer from type I allergic reactions. The symptoms are due to release of mediators (e.g. histamine) resulting from crosslinking of effector cell-bound IgE (immunoglobulin E) antibodies by allergens. The symptoms of type I allergy can be suppressed by various pharmacologic treatments, but allergen-specific immunotherapy (SIT) represents the only curative approach. A rise in allergen-specific IgG antibodies, particularly of the IgG4 class (Reid et al., J. Allergy Clin. Immunol. 78: 590-600, 1986), a reduction in the number of mast cells and eosinophils, and a decreased release of mediators (Varney et al., J. Clin. Invest. 92: 644-651, 1993) were found to be associated with successful SIT. Induction of specific anergy in peripheral T cells by IL-10 and subsequent reactivation of distinct cytokine patterns by cytokines from the tissue microenvironment proved to be key events in the immunologic mechanisms of SIT (Akdis and Blaser, Allergy 55: 522-530, 2000). In contrast to the peripheral anergy of specific T cells, the capacity of B cells to produce specific IgE and IgG4 antibodies is not affected by the treatment. In fact, an increase of the specific serum levels of both isotypes can be observed during the early phase of immunotherapy, although the increase in specific IgG4 is more pronounced. If the specific immunotherapy is successful, the ratio of specific IgE to IgG4 decreases significantly within a few weeks of treatment. Allergen-specific immunotherapy can induce specific regulatory T-cells (Treg) that abolish allergen-induced proliferation of T helper 1 (Th1) and Th2 cells, as well as their cytokine production. Treg cells utilize multiple suppressive mechanisms, interleukin-10 (IL-10) and transforming growth factor-β, mIL-10, TGF-βR and IL-10R as surface molecules. An important aspect of Treg cells is the regulation of antibody isotypes and suppression of pro-inflammatory cells. IL-10 and TGF-β secreted by Treg cells skew production of IgE towards the non-inflammatory isotypes, IgG4 and IgA, respectively. Furthermore, Treg cells may directly or indirectly suppress effector cells of allergic inflammation such as basophils and eosinophils. The induction and increasing secretion of IL-10 during SIT appears to counter-regulate allergen-specific IgE and IgG4 antibody synthesis. By suppressing both total and allergen-specific IgE and enhancing IgG4-synthesis, IL-10 shifts the allergic response from an IgE- to an IgG4-dominated phenotype (Akdis et al., J. Clin. Invest. 102: 98-106, 1998).

However, there are serious limitations to the use of currently available allergen preparations for specific immunotherapy. While multiple studies have demonstrated that successful SIT requires administration of high doses of allergens, effective dosages are limited by potential systemic reactions. As a result, specific immuntherapy usually requires a treatment period of 2 to 3 years, over which the allergen preparation is administered at slowly increasing dosages followed by several injections of the final maintenance dose. Since the compliance of patients and doctors is very low due to the tedious and and potentially harmful procedure, there is a need in the field for modified allergens capable of providing protection without the danger of serious side-effects.

Various attempts have been made to increase the success of SIT and decrease the risk of side-effects by allergen modification. The aim of such allergen modification is to decrease the allergenicity while retaining its immunogenicity. Since allergenicity depends on the interaction of a multivalent allergen with basophil- or mast cell-bound IgE antibodies, allergenicity can be reduced by decreasing the B cell epitope density of allergens. One approach is by partial or complete denaturation of allergens on chemical modification because the vast majority of B cell epitopes are of the discontinuous type, being dependent on the native conformation of proteins. However, there are serious limitations to the use of such molecules. While linear T cell epitopes are preserved, the surface structure is not maintained and, thereby, the capacity of such molecules to stimulate an allergen-specific non-IgE antibody response is severely limited. Another approach to reduce the accessibility of B cell epitopes is by polymerization via formaldehyde or glutaraldehyde treatment or by attachment of nonimmunogenic polymers. However, one limitation of this approach is that the loss of allergenicity by this technology is associated with a corresponding loss of other B cell epitopes. As a result, such molecules display a significantly reduced ability to induce IgG antibodies reactive with the natural allergen.

Recently, the molecular characterization of allergens by recombinant DNA technology has provided well defined allergens which allow the determination of their three-dimensional structure and site-directed modifications of their surface structure. Unfortunately, however, knowledge of IgE-specific B cell epitopes is scarce and useful tools for reliabe identification of such epitopes are not available. While several IgE epitopes could be determined by mapping with synthetic overlapping peptides synthesized according to the amino acid sequence of the allergen (van Hof et al., Mol. Immunol 28, 1225-1232, 1991; Ball et al., Clin Exp Allergy 24, 758-764, 1994), many relevant IgE epitopes could not be identified because peptides frequently fail to display conformations mimicking discontinuous epitopes. Similar considerations apply to the mimotope strategy utilizing combinatorial peptide libraries for identification of IgE epitopes (Gershoni et al., Immunol Today 18, 108-110, 1997). Screening of epitope libraries constructed from randomly DNAse I-digested allergen cDNA fragments turned out to yield more information than peptide mapping (Ball et al., J Biol Chem 269, 28323-28328, 1994; Ong et al., Mol Immunol 32, 295-302, 1995). However, the ability of polypeptides derived from randomly DNAse I-digested allergen cDNA, to generate discontinuous IgE epitopes is also limited since discontinuous epitopes are composed of at least two different areas of the molecule brought together by folding.

Monoclonal allergen-specific antibodies can be useful to identify IgE epitopes. For example, from a panel of mouse monoclonal antibodies that effectively inhibited binding of birch pollen allergen Bet v 1 to specific IgE, several monoclonal antibodies identified a continuous epitope within an exposed surface area of Bet v 1 that could be part of a discontinuous IgE epitope (Lebecque et al., J Allergy Clin Immunol 99, 374-384, 1997). Provided such antibodies bind to Bet v 1 with high affinity, they represent useful tools for further structural analyses by X-ray diffraction of crystals obtained from allergen-antibody complexes. Although the surface area recognized by these animal-derived allergen-specific antibodies may not be identical with that recognized by human IgE antibodies, both areas are closely related as indicated by the inhibition experiments. Therefore, structural information obtained from the analysis of such allergen-antibody complexes provide a valuable basis for the modification of IgE epotopes by site-directed mutagenesis.

One problem of this approach, however, is the need of a panel of high affinity antibodies with different epitope specificities for each allergen to allow for a detailed analysis of the total spectrum of potential IgE epitopes. Assuming that a B cell epitope takes up an area of approximately 900 A², the vast majority of allergens is likely to display more than one IgE epitope, in the case of allergens with a molecular weight of >40 kd most likely several IgE epitopes. Considering the poor immunogenicity of allergens, there is a need in the field to develop novel technologies for the generation of high affinity allergen-specific antibody panels that are capable of inhibiting human IgE binding.

There is no doubt that naturally occurring IgE antibodies represent ideal tools for structural analyses of IgE epitopes. However, the number of monoclonal allergen-specific IgE antibodies isolated from blood lymphocytes of allergic patients so far is extremely limited. Human IgE Fabs (fragment antigen binding) with specificity for the major timothy grass pollen allergen Phl p 5 were isolated from an IgE combinatorial library prepared from the mRNA of peripheral blood lymphocytes of a grass pollen allergic patient (Steinberger et al., J. Biol. Chem. 271: 10967-10972, 1996). cDNA encoding for IgE heavy chain fragments and for light chains was obtained by reverse transcription and PCR amplification, randomly combined in a suitable vector, and expressed on the surface of filamentous phage to allow the selection of IgE Fab-expressing phage clones by panning against immobilized Phl p 5. Using a similar approach, human IgE Fabs displaying specificity for the egg allergen ovomucoid and cross-reactivity for skim milk and bovine serum albumin were isolated from a combinatorial antibody library constructed from the mRNA of peripheral blood lymphocytes of an adult patient with atopic dermatitis (Edwards et al., J. Immunol. 168: 6305-6313, 2002). In another recent study, human IgE antibody fragments with specificity for hevein, the major latex allergen, were isolated from a phage library prepared from the mRNA of peripheral blood lymphocytes of a latex allergic patient (Laukkanen et al., J. Immunol Methods 251: 123-135, 2003). In this study, an scFv phage library constructed by combining IgE heavy chain genes with kappa and lambda light chain genes, was used, since the functionality of phage libraries displaying Fab antibody fragments proved to be poor resulting in low enrichment factors and unstable phagemid DNAs.

The few human monoclonal IgE antibodies with a defined allergen specificity isolated so far, emphasize the extreme difficulties associated with cloning and selecting allergen-specific IgE antibodies from the immune repertoire of peripheral blood mononuclear cells of allergic donors. The low number of IgE-secreting B-cells in the peripheral blood of allergic patients (MacKenzie and Dosch, J. Exp. Med. 169: 407-430, 1989) seriously hampers this approach for generating IgE reagents. Furthermore, it has been impossible so far to immortalize human B-lymphocytes that were switched to specific IgE production *in vivo* (Steinberger et al., J. Biol. Chem. 271: 10967-10972, 1996).

Another serious problem associated with the design of hypoallergenic molecules for an improved immunotherapy is the lack of understanding of the immune response that guarentees a lasting protection after specific immunotherapy. The aim to decrease the allergenicity of a given allergen while retaining its immunogenicity is widely accepted, but the term immunogenicity remains to be defined. Evaluation of modified recombinant allergens with a strongly reduced IgE reactivity that display the full spectrum of linear T cell epitopes but a different surface structure as compared to the corresponding natural allergen, have demonstrated that such molecules are capable of reducing specific IgE development towards the native allergen (Niederberger et al., Proc Natl Acad Sci USA 101, 14677-14682, 2004; Karamloo et al., Eur J Immunol 35, 3268-3276, 2005). However, a long lasting protective effect after treatment with these molecules has not been demonstrated. Apparently, the capacity of recombinant allergens to stimulate a long lasting protective allergen-specific non-IgE antibody response requires also a surface structure that is closely related to that of the corresponding natural allergen. Since disruption of IgE epitopes is associated with a significant alteration of the surface structure, there is a need in the field to identify those surface structures of allergens that mediate an appropriate non-IgE responsé for a long lasting protection.

### THE PRESENT INVENTION

This need is met by the subject matter of the claims. In particular, the present invention provides a method of obtaining a composition comprising recombinant antibodies, wherein the antibodies in the composition are capable of binding to all epitopes of an allergen recognized by antibodies of a specific isotype, in particular either human IgE, IgG4 or IgA antibodies, such a composition and the use of individual antibodies of such a composition as tools for the design of hypoallergenic molecules for specific immunotherapy, as well as a method for producing hypoallergenic molecules for specific immunotherapy.

In a preferred embodiment, the invention provides a method for generating a composition comprising antibodies capable of binding to all epitopes on an allergen X recognized by antibodies of one or more specific isotype(s), comprising steps of
a) generating a plurality of allergen-specific antibodies capable of binding to the allergen X,
b) combining a plurality of the generated antibodies and testing whether essentially complete inhibition of binding of the allergen X to human IgE, IgG4 and/or IgA antibodies in a pool serum of patients allergic to said allergen or obtained from said serum is achieved,
c) in case essentially complete inhibition is not achieved in step b), repeating steps a) and b);
d) in case essentially complete inhibition is achieved, reducing the number of antibodies to the minimal number of antibodies sufficient for essentially complete inhibition by a method wherein
   i) groups of the antibodies obtained in step a) are generated, comprising different numbers and combinations of antibodies;
   ii) said groups are tested for essentially complete inhibition of binding of the allergen X to IgE, IgG4 and/or IgA antibodies in a pool serum of patients allergic to said allergen or obtained from said sera;
   iii) wherein, in case one or more group effects essentially complete inhibition in step ii), steps i) and ii) are repeated with subcombinations of the antibodies from said group or groups until the minimal number of antibodies in said group or groups is identified which effects essentially complete inhibition;
   iv) wherein, in case essentially complete inhibition is not achieved in step ii) or iii), steps i), ii) and iii) are repeated with different groups of antibodies;
and wherein the group identified in step d), iii) is said composition.

Preferably, the composition comprises antibodies capable of binding to all epitopes recognized by IgE, IgG4 and/or IgA antibodies, in particular, by human IgE, IgG4 and/or IgA antibodies. It is preferred that the composition comprises the minimal number of antibodies necessary and sufficient for binding to all epitopes recognized by human IgE antibodies on an allergen X. Additional antibodies may, however, be added.

In the context of this invention, the term "epitope recognized by human IgE (IgE epitope), IgG4 (IgG4 epitope) or IgA antibodies (IgA epitope)", relates to the surface area of an allergen that is in contact to these antibodies upon binding to the allergen. It also relates to the surface area of the allergen that is in contact with an antibody construct comprised in the composition of the invention, that overlaps with the first-mentioned IgE epitope, IgG4 epitope or IgA epitope, so binding of the antibody construct can inhibit binding of the human IgE, IgG4 or IgA antibodies from the sera of patients allergic to the allergen (IgE related epitopes, IgG4 related epitopes, IgA related epitopes). Preferably, the epitopes overlap by 20% or more, 50% or more, 60% or more, 70% or more, or 80% or more. Most preferably, the epitopes overlap by 90 or 95% or more or are identical. With reference to the number of epitopes of an allergen, the first-mentioned epitopes and the related epitopes are considered to represent the same epitopes.

The allergen X can be any allergen, in particular, one of the allergens listed in www.allergen.org or www.allergome.org. For example, the allergen X can be selected from a group comprising api m 1, api m2, api m 3, api m 4 and api m5. Importantly, the allergen X is the same allergen throughout the method of the invention. If not mentioned otherwise, "allergen" refers to the allergen X. Thus, for example, the patients from whom the pool serum is derived are allergic to the same allergen that the generated antibodies bind to or for which inhibition of binding is tested. In particular, the term allergen X refers to an allergen that has been prepared by a reproducible method from a reproducible source, and the allergen used throughout the method of the invention has been prepared by the same method. Preferably, the allergen is a recombinant allergen, as it doesn't depend, e.g., on variations of the source material, and can be produced in unlimited amounts. It is preferred that the recombinant allergen has been found to be essentially functionally identical to the same allergen purified from natural sources, e.g., in skin prick tests of different patients allergic to the allergen. The allergen can also be prepared from natural sources.

The plurality of allergen-specific monoclonal antibodies can be generated from different sources. Naturally occurring IgE, IgG4 and IgA antibodies represent ideal tools for structural analyses of IgE, IgG4 and IgA epitopes, but their availability is limited. Cloning and selecting allergen-specific IgE antibodies from the immune repertoire of peripheral blood mononuclear cells of allergic donors is extremely difficult. The low number of IgE-secreting B cells in the peripheral blood of allergic patients (MacKenzie and Dosch, J. Exp. Med. 169: 407-430, 1989) seriously hampers this approach for generating monoclonal IgE antibodies. Cloning and selecting allergen-specific IgG4 antibodies from the immune repertoire of peripheral mononuclear cells of allergic donors may be less difficult due to the significantly higher number of IgG4-secreting B cells in the peripheral blood of allergic patients as compared to IgE-secreting B cells. Currently, however, the availability of human monoclonal allergen-specific IgG4 antibodies is limited. Similar considerations apply to the generation of human monoclonal allergen-specific IgA antibodies.

Preferably, the antibodies in the composition obtained by the method of the invention are recombinant antibodies. Semisynthetic or synthetic antibody libraries (e.g., scFv or Fab format) provide a high degree of variability and, thereby, represent a valuable alternative for generating the required plurality of allergen-specific monoclonal antibody fragments. However, newly generated antibody libraries derived from animals (mammalian species as well as avian species) after immunization with the allergen X provide a significantly higher number of allergen-specific variable antibody domains and, thereby, an increased probability for the selection of the required plurality of allergen-specific high affinity monoclonal antibody fragments. In a preferred embodiment a combination of antibody libraries derived from avian and mammalian species after immunization with the allergen X are used. The phylogenetic difference between avian and mammalian species provides access to different antibody repertoires. IgY antibodies recognize other epitopes than mammalian antibodies. Therefore, a combination of antibody libraries from avian and mammalian species provides a significant advantage for generating a plurality of allergen-specific high affinity monoclonal antibodies. If it should - unexpectedly - be found that the combination of all antibodies capable of binding to the allergen is not sufficient to effect essentially complete inhibition of binding of the allergen X to antibodies in a pool serum of patients allergic to said allergen or obtained from said sera, it is recommended to additionally use further antibodies from a different library. Methods for generating antibody libraries are known in the art (e.g., Steinberger et al., J Biol Chem 271: 10967-10972, 1996; Edwards et al., J Immunol 168: 6305-6313, 2002; Laukkanen et al., J Immunol Methods 251: 123-135, 2003; Boel et al., J Immunol Meth 26: 153-166, 2000).

Preferably, the plurality of antibodies is 2 to 2000 antibodies, more preferably, 2 to 200 antibodies or 2 to 20 antibodies.

Generally, in the context of the invention, the term antibodies comprises natural antibodies (originating from natural sources) as well as in recombinant form as well as antibody constructs, such as scFv or Fab antibodies. Preferably, the recombinant antibodies in the composition are scFv or Fab antibodies.

In the method of the invention, inhibition of binding of the allergen X to IgE can be determined by incubating IgE antibodies in a pool serum of patients allergic to the allergen or antibodies obtained from said serum with human basophils after stripping of said basophils, and with or without preincubation of allergen X with the recombinant antibodies or recombinant antibody fragments or, for comparison, antibodies in the pool serum of patients allergic to said allergen or obtained from said serum, contacting said basophils with said allergen, and detecting release of histamine or another marker molecule.

Alternatively, inhibition can be determined by contacting, e.g., anti IgE antibodies immobilized on a carrier with antibodies in a pool serum of patients allergic to the allergen X or obtained from said serum, and, with or without preincubation of labelled allergen X with the recombinant antibodies or recombinant antibody fragments or, for comparison, antibodies in a pool serum of patients allergic to said allergen or obtained from said serum, contacting the carrier with said allergen X and detecting binding of the labelled allergen X to the carrier. For this purpose, the allergen X can be labelled with an enzyme, a radioisotope, biotin or a fluorescent marker.

According to this method, inhibition of binding of the allergen X to IgG4 and IgA can also be determined.

The inhibition is considered essentially complete if it is comparable to the inhibition by IgE, IgG4 or IgA antibodies in a pool serum of patients allergic to said allergen or obtained from said serum, i.e. if it varies from that inhibition by about 20% or less, preferably by about 10% or less, or most preferably, by about 5% or less.

The pool serum used in the present invention comprises serum from several patients allergic to the allergen X. Preferably, said pool serum comprises the antibodies from the sera of at least 5 patients, more preferably at least 10 patients or most preferably at least 15 patients allergic to said allergen. For IgE inhibition experiments, patients are preferred that are highly sensitized to the allergen. For IgG4 and IgA inhibition experiments, patients after successful SIT are preferred.

IgE antibodies can be obtained from the pool serum, e.g., by affinity chromatography using anti-human IgE antibodies. Preferably, IgG antibodies are removed from the pool serum, e.g. by pre-treatment with a protein A matrix, such as a protein A column. This step, however, is not essential, as sera of allergic patients in all probability only contain relatively low amounts of allergen-specific IgG antibodies (Ganglberger et al., FASEB J. 14:2177-2184, 2000), even though the serum level of IgG is about 10000 times higher than the serum level of IgE. IgG4 as well as IgA antibodies can also be obtained from the pool serum by affinity chromatography using anti-human IgG4 or anti-human IgA antibodies.

The invention also relates to the compositions of antibodies obtainable by the method of the invention, which comprise a plurality of recombinant antibodies, wherein the antibodies in the composition are capable of binding to all epitopes of an allergen X recognized by antibodies of one or more specific isotype(s). The preferred antibody compositions of the invention comprise 2 or more antibodies, 3 or more antibodies, 4 or more antibodies or 5 or more antibodies.

For an estimation of the number of antibodies sufficient for binding to all epitopes recognized by human IgE, IgG4 and/or IgA antibodies on a particular allergen, it is important to know the approximate number of possible B cell epitopes per allergen. Therefore, methods for estimating the number of B cell epitopes per allergen have been developed. These methods are based on the following parameters:
a) Calculation of the surface of structurally characterized allergens in A²: The solvent accessible surfaces of proteins can be calculated with the aid of POPS (parameter optimized surfaces) according to Fratemali F. et al. (Nucleic acids Res. 30:2950-2960, 2002).
b) Surface area of B-cell epitopes in A²: At the moment, one co-crystallization of allergen and antibody is available only, namely for the allergen Bet v 1 and a murine allergen-specific Fab-fragment. The surface area of this discontinuous epitope is 931 A² (Mirza et al., J. Immunol. 165:331-338, 2000). This correlates well with the area of other B cell epitopes (circa 2x3 nm).

In table 1, the surface of allergens for which structural data is available in the protein data bank (PDB) was calculated with the aid of a molecule of water. Under the assumption that a B cell epitope takes up an area of 950 A², the maximal possible number of B cell epitopes for an allergen (without differentiation for IgE, IgG or IgA epitopes) was determined. The number calculated in this way is much too high, but can be considered to provide an upper limit for the number of necessary antibody constructs for an allergen. On the basis of this data, an approximate relation between molecular weight and potential B cell epitopes was calculated. The mean value of the upper limit for potential B cell epitopes is approx. 0.5 B cell epitopes per 1 kDa.

Table 2 summarizes allergens that have been examined for IgE binding structures with overlapping oligopeptides. Utilizing overlapping oligopeptides (e.g., decapeptides), more potential IgE epitopes are identified than exist in reality, as the majority of IgE epitopes are discontinuous epitopes composed of at least two different areas of the molecule brought together by folding. Different relevant allergens, such as Phospholipase A2 and the birch pollen allergens Bet v1, Bet v3 and Bet v4 exclusively have discontinuous epitopes (Valenta et al., Int. Arch. Immunol. 116, 167-176, 1998). Since the identified linear epitopes probably only form part of these discontinuous epitopes, for estimation of the number of epitopes it is supposed that at least three linear IgE binding epitopes are, as partial structures, involved in forming a discontinuous IgE epitope.

In table 2, the number of identified IgE binding peptides has been divided by three and related to the molecular weight of the allergen. A number of 0,06 to 0,19 epitopes per 1 kDa calculated on the basis of linear IgE binding peptides is preferred. The best estimation is possible on the basis of the number of 0,12 IgE epitopes per 1 kDa, which is possibly still too high but could be considered realistic.

As a result, the preferred antibody compositions of the invention comprise
- approx. 1 to 2 antibodies or antibody fragments for an allergen X having a molecular weight of less than 10 kDa,
- approx. 1 to 3 antibodies or antibody fragments for an allergen X having a molecular weight of 10 to 20 kDa,
- approx. 2 to 4 antibodies or antibody fragments for an allergen X having a molecular weight of more than 20 to 30 kDa,
- approx. 3 to 5 antibodies or antibody fragments for an allergen X having a molecular weight of more than 30 to 40 kDa,
- approx. 4 to 7 antibodies or antibody fragments for an allergen X having a molecular weight of more than 40 to 50 kDa,
- approx. 5 to 8 antibodies or antibody fragments for an allergen X having a molecular weight of more than 50 to 60 kDa,
- and approx. 6 to 19 antibodies or antibody fragments for an allergen X having a molecular weight of approximately 100 kDa for an allergen X with a higher molecular weight.

The preferred number of antibodies correlates well with known data for Bet v 2 (17.4 kDa), which can be bound by three different monoclonal Fab fragments (without differentiating between IgG and IgE epitopes, Valenta R. et al., Int. Arch. Immunol. 116, 167-176, 1998). Bet v 2 has at least two IgE epitopes, since it can induce *in vivo* cross-linking of surface bound IgE antibodies.

Although no data are available for estimation of the number of antibodies or antibody fragments in compositions for identification of IgG4 and IgA epitopes, such compositions can be assumed to comprise a comparable number of antibodies or antibody fragments. Considering 0.5 B cell epitope per 1 kDa as upper limit, 0.1 IgG4 epitope per 1 kDa as well as 0.1 IgA epitope per 1 kDa appears to be a reasonable assumption, although possibly still too high. The assumed similar density of IgG4 and IgA epitopes as compared to that of IgE epitopes translates into a comparable number of antibodies or antibody fragments.

In one aspect, the present invention provides a method of identifying all epitopes on an allergen X recognized by antibodies of one or more specific isotype(s), comprising carrying out the methods of the invention described above, and identifying the epitopes recognized by the antibodies in the obtained composition. Thus, the composition of antibodies of the invention is used for identifying all epitopes of the allergen recognized by antibodies of one or more specific isotype(s).

Preferably, the individual antibodies of a generated composition are used for structural analyses of IgE, IgG4 or IgA epitopes. Since each composition effects essentially complete inhibition of binding of the allergen X to patient-derived IgE, IgG4 or IgA antibodies, the individual antibodies of each generated composition are capable of identifying all epitopes on allergen X that are accessible for patient-derived IgE, IgG4 or IgA antibodies.

The epitopes can be identified, e.g., by X-ray structure analysis of complexes of the individual antibody/allergen complex, or by testing binding of antibody after site-directed mutagenesis of the allergen.

In particular, antibody compositions capable of binding to all epitopes of a particular allergen that are recognized by human IgE antibodies, are utilized to identify and modify those amino acid residues involved in the interaction with allergen-specific human IgE antibodies by site-directed mutagenesis, thereby eliminating or decreasing the allergenicity of a particular allergen in a structure-based approach. By site-directed mutagenesis of amino acid residues essential for the allergen-IgE antibody interaction, IgE epitopes are eliminated with minimal impairment of the residual surface structure important for a non-IgE immunological response.

Accordingly, a method of generating a hypoallergenic molecule for specific immunotherapy is presented, comprising identifying all epitopes recognized by IgE antibodies according to the methods of the inventions, and modifying amino acid residues of the epitopes identified thereby to reduce recognition of the hypoallergenic molecule by the IgE antibodies. Preferably, furthermore, all epitopes recognized by IgG4and/or IgA antibodies are identified according to the methods of the inventions, and amino acids of the epitopes identified thereby are maintained to maintain recognition of the hypoallergenic molecule by the IgG4 and/or IgA antibodies.

Antibody compositions capable of binding to all epitopes of a particular allergen that are recognized by human IgG4 and/or IgA antibodies, are utilized to maintain those structures that mediate an appropriate non-IgE response for a long lasting protection after specific immunotherapy. This rational is based on the recent observation that immune deviation towards T regulatory (Treg) cells is an essential step in successful SIT (for a review, see Jutel et al., Allergy 61, 796-807, 2006). Treg cells are defined by their ability to produce high levels of IL-10 and TGF-β and to suppress naive and memory T helper type 1 and 2 responses. There is now clear evidence that IL-10- and/or TGF-β-producing T regulatory cells are generated in humans during the early course of SIT. Since Treg cells have been shown to differentiate from naive T cells in the periphery upon encountering antigens present at high concentrations, it can be assumed that Treg cells are also induced by high and increasing doses of allergens. Most important is the fact that IL-10 and TGF-β directly or indirectly suppress effector cells of allergic inflammation such as basophils and mast cells, and induce IgG4 and IgA production, respectively, from B cells as non-inflammatory immunoglobulin isotypes and suppress IgE production. Based on these observations, antibody compositions capable of binding to all epitopes of a particular allergen that are recognized by human IgA and IgG4 antibodies are utilized to identify and maintain those amino acid residues involved in the interaction with allergen-specific human IgA and IgG4 antibodies (for allergens entering the body via the mucosa, e.g. inhalation allergens). With regard to those allergens that do not enter the body via the mucosa such as insect venom allergens, antibody compositions capable of binding to all epitopes recognized by human IgG4 antibodies are utilized to identify and maintain those amino acid residues involved in the interaction with allergen-specific human IgG4 antibodies.

Accordingly, the invention also provides an hypoallergenic molecule, which can be advantageously used for specific immunotherapy. The most potent hypoallergenic molecule for specific immunotherapy is an allergen that does not exhibit allergenicity, contains an array of T cell epitopes that is comparable to that of the corresponding natural allergen, and displays a surface structure that is recognized by human IgA and/or IgG4 antibodies with specificity for the corresponding natural allergen. For the design of such a molecule, the individual antibodies of the different antibody compositions are essential to maintain IgA and/or IgG4 epitopes upon modification of the IgE epitopes by a structure-based approach.

In a preferred embodiment, allergenicity is reduced to 20% while at least 50% of IgA and/or IgG4 epitopes are maintained. In a more preferred embodiment, allergenicity is reduced to 10% while at least 50% of IgA and/or IgG4 epitopes are maintained. In a most preferred embodiment, allergenicity is reduced to <5% while at least 50% of IgA and/or IgG4 epitopes are maintained.

In the context of this invention, allergenicity is defined as the capability of a proteinaceous allergen to bind human IgE antibodies. Antigenicity in the context i of this invention is defined by the capability of a proteinaceous allergen to bind human IgG4 and/or IgA antibodies.

### SPECIFIC EXAMPLES

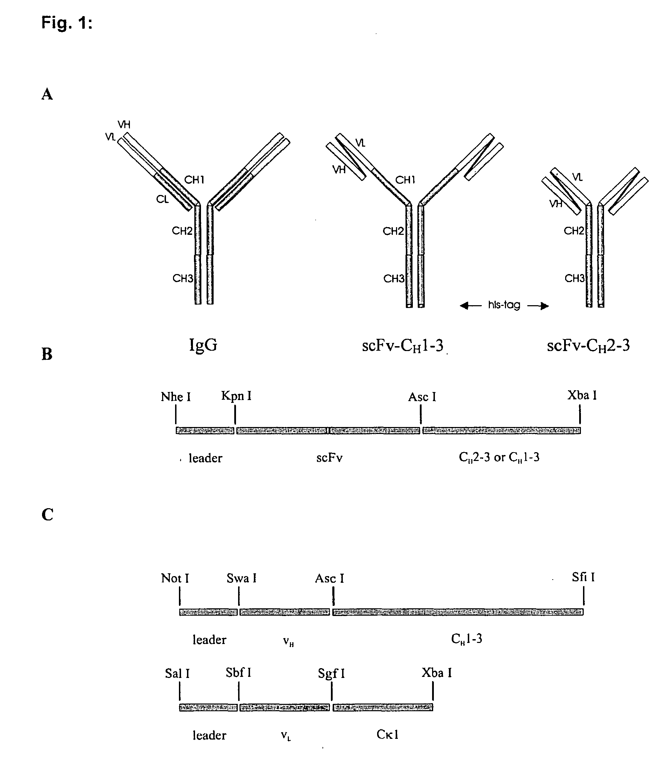

A variety of antibody formats including scFv-derived constructs as well as complete antibody constructs are suitable for the present invention. The structure of some of these antibody formats described in the following examples are shown in Figure 1.

### EXAMPLE 1: CONSTRUCTION OF AN IMMUNE AVIAN scFv PHAGEMID LIBRARY

One adult female White Leghorn chicken (designated C160) was immunized with bee venom from *Apis mellifera* (Latoxan, Valence, France). C160 received three intradermal allergen doses at 21-day intervals, containing each 50 mg of hymenoptera venom in a total volume of 400 mL. The first dose was prepared with complete Freund's adjuvant and the two subsequent doses were administered with incomplete Freund's adjuvant. On days 6 and 7 post injection, eggs were collected and their yolks were processed using thiophilic adsorption chromatography according to the manufacturer's recommendations (Amersham Biosciences, Germany) to monitor polyclonal antibody response. Venom-specific reactivity of purified IgY was verified using AlaBLOTs I1 (DPC Biermann, Bad Nauheim, Germany) according to the manufacturer's recommendations.

### RNA isolation

Five days following the final immunization, the animals were euthanized, exsanguinated, and the spleens were harvested. 50-100 mg of the spleen from an individual animal were placed immediately in 1 ml of TRIFast reagent (PeqLab, Erlangen, Germany). This is a monophasic solution containing phenol and guanidine thiocyanate in which the tissue was homogenized. The homogenate was centrifuged for 10 min at 2500 g. The supernatant was transferred to a clean tube, avoiding the fatty layer and pellet that separate upon centrifugation. Phase separation was carried out by addition of 200 µl chloroform per 1 ml TriFAST reagent followed by vortexing and incubation at room temperature for 15 min. The homogenate was centrifuged at 10000 *g* for 15 min at 4°C. The clear, aqueous phase was transferred to a clean tube and 15 ml of isopropanol were added. The tube was vortex-mixed, incubated at room temperature for 10 min, and centrifuged at 10000 *g* for 30 min at 4°C to pellet the RNA. The supernatant was discarded and the pellet was washed with 75% ethanol and centrifuged a second time at 10000 g for 30 min at 4°C. The pellets were air-dried and resuspended in 50 µl of nuclease-free water. The concentration and purity were determined by measuring the absorbance (A) at 260 nm and 280 nm. The RNA was stored at -80°C.

### cDNA Synthesis

First-strand cDNA was synthesized using Superscript III reverse Transcriptase from Invitrogen (Germany). For this, 5 µg of total RNA was mixed with 4 µl deoxynucleotide mix (10 mM), 5 pmol gene-specific primers (avian Cυ1-specific antisense primers), and water to a final volume of 13 µl and heated for 5 min at 65°C. Subsequently, 4 µl of 5x reaction buffer, 1 µl Ribolock RNAse inhibitor (Fermentas, Lithuania) and 1 µl superscript III were added. The reaction mixture was incubated for 60 min at 55°C. The reaction was then terminated by heating to 100°C for 5 min, followed by a 5-min incubation on ice. The first-strand cDNA was directly used for PCR amplification.

### Generation of scFv phagemid libraries

First-strand cDNA was used to amplify VH and VL genes for the construction of combinatorial scFv antibody libraries. In order to generate scFv fragments two PCR steps are required - a primary PCR which amplifies VH and VL gene segments separately and a secondary overlap PCR which randomly combines VH and VL via a common linker sequence (GGSSRSSSSGGGGSGGGG, SEQ ID NO: 51) to form a full-length scFv fragment.

The avian Fd region was amplified by PCR using primers specific for the variable regions of the heavy chain (gccgtgacgttggacgag, SEQ ID NO: 41 and ggaggagacgatgacttcggtcc, SEQ ID NO: 42). Accordingly, the light chains were amplified using specific primers for variable region of the light chains (ctgactcagccgtcctcggtgtc, SEQ ID NO: 43 and taggacggtcaggccagagccac, SEQ ID NO: 44).

In each case 1 µl of cDNA was mixed with 60 pmol of each primer, 10 µl of 10x reaction buffer AccuPrime I, 1 unit Taq High Fidelity DNA polymerase (Invitrogen, Karlsruhe, Germany), and water to a final volume of 50 µl. The reactions were carried out under the following conditions in an thermal cycler (MasterCycler, Eppendorf, Germany): 30 cycles of 94°C for 15 s, 56°C for 30 s, and 72°C for 1.5 min, followed by a final extension at 72°C for 10 min. The 350-400 base pair (bp) fragments were size selected on a 1% agarose gel and purified from the gel slice using a gel extraction kit (PeqLAb, Erlangen, Germany). The secondary overlap PCR fragment was amplified using 100 ng of each appropriate primary PCR product in conjunction with the sense primer and the antisense primer. These oligonucleotide primers have the Sfi I and Not I cloning sites incorporated into the sequence for subsequent cloning into the phagemid vector. The remaining components were present in the same concentration as described above. The number of cycles was decreased to 25 and the extension time was increased to 2 min, while the remaining conditions were the same as those described above. The 750-800 bp products were size-selected and purified as discussed above in preparation for restriction endonuclease digestion and inserted into pHen2 phagemid vector containing the ccdB gene for negative selection via Sfi I and Not I sites.

### EXAMPLE 2: CONSTRUCTION OF HUMAN IgE, IgA AND IgG4 ANTIBODY LIBRARIES

### RNA isolation

50 ml of heparinised blood were obtained from patients suffering from type I hypersensitivity reactions. Lymphocytes were isolated employing histopaque-ficoll density gradient centrifugation (Sigma Aldrich). Subsequently, lymphocytes were enriched for B cells and plasma cells by MACS-sorting using an anti-CD19-antibody (Miltenyi Biotech). Lymphocyte RNAs were isolated using 1 ml TriFast reagent (PeqLab, Erlangen, Germany) per 5-10 x 10⁶ lymphocytes. Total RNA Isolation was performed as mentioned above.

### cDNA Synthesis

First-strand cDNA was synthesized using Superscript III reverse Transcriptase from Invitrogen (Germany). For this, 5 µg of total RNA was mixed with 4 µl deoxynucleotide mix (10 mM), 5 pmol gene-specific primers (human Cε1, Cα1 or Cγ₄1-specific antisense primers), and water to a final volume of 13 µl and heated for 5 min at 65°C. Subsequently, 4 µl of 5x reaction buffer, 1 µl Ribolock RNAse inhibitor (Fermentas, Lithuania) and 1 µl superscript III were added. The reaction mixture was incubated for 60 min at 55°C. The reaction was then terminated by heating to 100°C for 5 min, followed by a 5-min incubation on ice. The first-strand cDNA was directly used for PCR amplification.

### Generation of scFv phagemid libraries

First-strand cDNA was used to amplify VH and VL genes for the construction of combinatorial scFv antibody libraries. In order to generate scFv fragments two PCR steps are required - a primary PCR which amplifies VH and VL gene segments separately and a secondary overlap PCR which randomly combines VH and VL via a common linker sequence (GGSSRSSSSGGGGSGGGG, SEQ ID NO: 51) to form a full-length scFv fragment.

The human Fd region was amplified by PCR using the primers specific for the variable regions of the heavy chains (VH1a-VH7a) and human JH primers. Accordingly, the kappa and lambda light chains were amplified using specific primers for variable region of the light chains (Vk1a-Vk6b and VL1a-VL10), Jκ and Jλ, respectively. The primer pools (VH1a-VH7a, Vk1a-Vk6b, JH and VL1a-VL10, Jκ and Jλ) were synthesized according to the PCR primers for the amplification of the functional human V genes listed in V Base directory (V Base Sequence Directory, Tomlinson et al., MRC Centre for Protein Engineering, Cambridge, UK).

In each case 1 µl of cDNA was mixed with 60 pmol of each primer, 10 µl of 10x reaction buffer AccuPrime I, 1 unit Taq High Fidelity DNA polymerase (Invitrogen, Karlsruhe, Germany), and water to a final volume of 50 µl. The reactions were carried out under the following conditions in a Eppendorf thermal cycler: 30 cycles of 94°C for 15 s, 56°C for 30 s, and 72°C for 1.5 min, followed by a final extension at 72°C for 10 min. The 350-400 base pair (bp) fragments were size selected on a 1% agarose gel and purified from the gel slice using a gel extraction kit (PeqLAb, Erlangen, Germany). The secondary overlap PCR fragment was amplified using 100 ng of each appropriate primary PCR product in conjunction with the sense primer and the antisense primer. These oligonucleotide primers have the Sfi I and Not I cloning sites incorporated into the sequence for subsequent cloning into the phagemid vector. The remaining components were present in the same concentration as described above. The number of cycles was decreased to 25 and the extension time was increased to 2 min, while the remaining conditions were the same as those described above. The 750-800 bp products were size-selected and purified as discussed above in preparation for restriction endonuclease digestion and inserted into pHen2 phagemid vector containing the ccdB gene for negative selection via Sfi I and Not I sites.

### EXAMPLE 3: GENERATION OF ALLERGEN-SPECIFIC scFv

### scFv (single chain fragment variable) phage display libraries

For selection of human recombinant antibody fragments with specificity for allergens the Griffin. 1 library was employed. This library is a scFv phagemid library based on synthetic V-gene segments of human origin. The library was built by recloning the heavy and light chain variable regions from the lox library vectors (Griffiths et al., EMBO J, 1994) into the phagemid vector pHEN2. The library was obtained from the MRC Center of Protein Engineering (Cambridge, UK).

For selection of donor-derived human scFv antibody fragments with specificity for allergens libraries were constructed on the basis of lymphocytes obtained from allergic patients.

For selection of avian recombinant antibody fragments with specificity for allergens avian immune libraries were constructed on the basis of spleen from immunized chicken as mentioned before and subjected to biopanning.

### Selection of the scFv phage display library

Purified allergens (e.g., bee venom phospholipase A2 (Latoxan, Valence, France); lysozyme (Sigma, Taufenstein, Germany); Phl p 5b (Biomay, Salzburg, Austria)) were diluted in 2 ml of phosphate buffered saline (PBS) to a final concentration of 100 µg/ml and used for coating of immunotubes (Maxisorp, Nunc, Wiesbaden, Germany) overnight at 4° C. The tubes were rinsed twice with PBS and blocked with PBS-milk powder (2% w/v, MPBS) for 2 h at room temperature. After removal of the blocking buffer, 10¹² to 10¹³ phages preincubated in 4 ml 2% w/v MPBS were added and incubated for 30 min at room temperature under continuous rotation, followed by another 90 min without rotation. Thereafter, the phage containing supernatant was discarded, the tube washed 10 times with 0.1% v/v Tween-PBS and another 10 times with PBS. The number of washing steps was increased to 25 times each during the selection procedure. Elution of bound phages was performed by adding 1 ml of 100 mM triethylamine and continuous rotation for 9 min. Eluted phages were removed and neutralized immediately by adding 0.5 ml 1M Tris-HCl, pH 7.4. Remaining phages in the immunotube were neutralized with 200 µl of 1M Tris-HCl, pH 7.4. Reinfection of an exponentially growing culture of *E. coli* TG1 was performed by incubation in the presence of the eluted phages for 30 min at 37° C without shaking. The infected *E. coli* TG1 culture was centrifuged at 3300 g for 10 min, then plated on tryptone yeast extract plates with 100 µg/ml ampicillin and 1% w/v glucose, and grown at 30° C overnight. After four rounds of selection for affinity to the specific allergens, single colonies were randomly picked for further characterization.

### Polyclonal and monoclonal phage ELISA

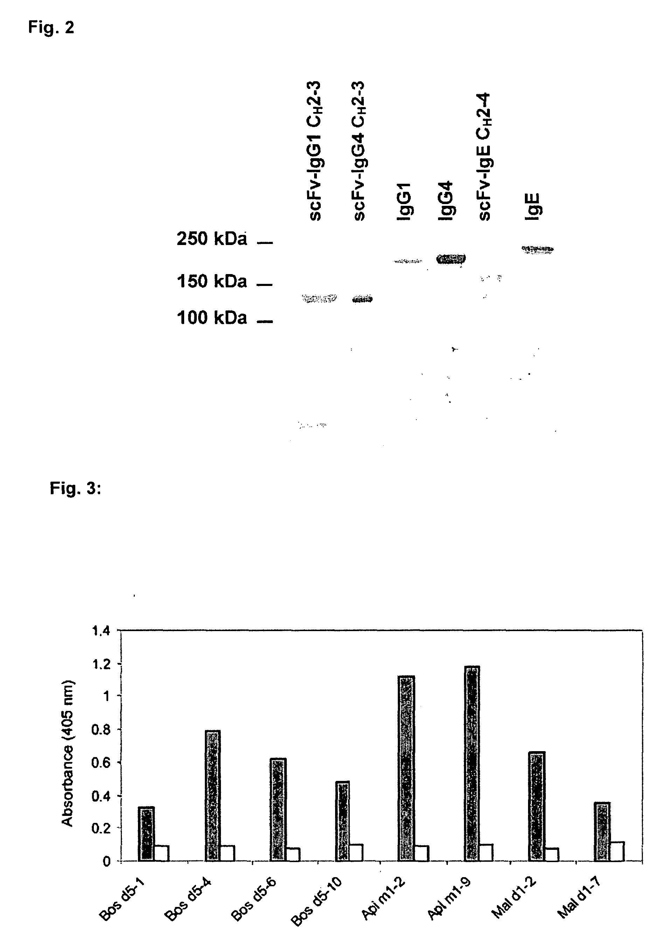

For assessment of immunoreactivity, 75 µl of phages (diluted 1:2 with 4% w/v MPBS) were applied to microtiter plates coated with the particular proteins (e.g. bee venom phospholipase A2 (Latoxan, Valence, France); lysozyme (Sigma, Taufenstein, Germany); Phl p 5b (Biomay, Salzburg, Austria)) at 4° C overnight, and blocked with MPBS at room temperature for 2 h. After incubation of the microtiter plates for 90 min at room temperature on a rocker platform, the wells were rinsed 3 times each with PBS-Tween (0.1% v/v, TPBS) and PBS and further incubated with 100 µl each of an anti-M13-HRP conjugate (Pharmacia Biotech, Freiburg, Germany) diluted 1: 5000 in 2% w/v MPBS for 60 min at room temperature on a rocker platform. The wells were rinsed again 3 times each with TPBS and PBS, and bound phages were visualized by the addition of 75 µl of a substrate solution (2,2-azino-bis(3-ethylbenzothiazoline-6-sulfonic acid) diammonium salt; ABTS; Sigma, Taufenstein, Germany). Absorbance was determined at 405 nm after 20 min of incubation. Examples of the immunoreactivity of selected monoclonal phages are shown in Figures 2 and 3, in Figure 2 the immunoreactivity of selected monoclonal phages after three rounds of selection against bee venom phospholipase A2 (PLA2), and in Figure 3 the immunoreactivity of selected monoclonal phages after three rounds of selection against the timothy grass (*Phleum pratense*) protein Phl p 5b.

### EXAMPLE 4: AMPLIFICATION AND CLONING OF scFv-DERIVED HOMODIMERIC CONSTRUCTS

### Amplification and cloning of human constant regions for scFv-derived constructs.

All human immunoglobulin constant domains were synthesized from cDNA derived from human peripheral mononuclear cells.

If not mentioned otherwise, the following protocol was used for PCR amplifications: 1x 1min 95°C, 30x (30 s 95°C,1 min 53°C, 1 min 72°C), 1x 5 min 72°C.

The human γ1C_{H}2-3 domain was amplified using one PCR primer containing a Xba I site (gatcggtaccgatcggcgcgcccaaatcttgtgacaaaactcac, SEQ ID NO: 1) and another primer containing an Asc I and a Kpn I site (gatctctagatcatttacccggagacagggagaggctcttc, SEQ ID NO: 2). The γ1 C_{H}1-3 domain was amplified using one PCR primer containing a Xba I site (gatcggtaccgatcggcgcgcccaaatcttgtgacaaaactcac, SEQ ID NO: 3) and another primer containing an Asc I site (gatcggcgcgccagcctccaccaagggcccat, SEQ ID NO: 4).

The human γ4C_{H}2-3 domain was amplified using one PCR primer containing a Xba I site (agtatctagatcatttacccagagacaggga, SEQ ID NO: 5) and another primer containing an Asc I site (gatcggcgcgcccagcaacaccaaggtggaca, SEQ ID NO: 6). The γ4C_{H}1-3 domain was amplified using one PCR primer containing a Xba I site (agtatctagatcatttacccagagacaggga, SEQ ID NO: 7) and another primer containing an Asc I site (gatcggcgcgccttccaccaagggcccatccgtcttccccct, SEQ ID NO: 8).

The human εC_{H}2-4 domain was amplified using one PCR primer containing a Xba I site and a 4xHis overhang (gatctctagatcaatggtggtgatgtttaccgggatttacagacaccg, SEQ ID NO: 9) and another primer containing an Asc I site (gatcggcgcgcccaccgtgaagatcttac, SEQ ID NO: 10). The human a1C_{H}1-3 domain and a2C_{H}1-3 domain was amplified using one PCR primer containing a Xba I site and a 4xHis overhang (gatctctagatcaatggtggtgatggtggacggcacctgctac, SEQ ID NO: 46) and another primer containing an Asc I site (gatcggcgcgcccgcatccccgaccagcc, SEQ ID NO: 45).

### Amplification and cloning of the rodent κ light chain leader sequence

The signal sequence of a rodent κ light chain was amplified assembled by overlapping PCR primer containing a Nhe I site (gtacaagcttgctagcaagatggaatcacagacccaggtcc, SEQ ID NO: 11) and another primer containing a Kpn I site (gtacacgcgttgtaaggactcaccccacaggtaccagaaa, SEQ ID NO: 12). After introduction of the κ light chain leader sequence into the mammalian expression vector pcDNA3.1-zeo (Invitrogen life technologies, Karlsruhe, Germany), the individual immunoglobulin Fc domains were inserted via the Xba I and the Asc I sites.

### Amplification and cloning of human J chain sequence

The human J chain was amplified from cDNA using a primer containing an Nhe I site (gatcgctagcatgaagaaccatttgcttttc, SEQ ID NO: 49) and another primer containing a Xba I site (gatctctagattagtcaggatagcaggcatc, SEQ ID NO: 50). The J chain sequence was then inserted into the mammalian expression vector pcDNA3.1-neo (Invitrogen life technologies, Karlsruhe, Germany).

### Amplification and cloning of scFv-constant domains constructs

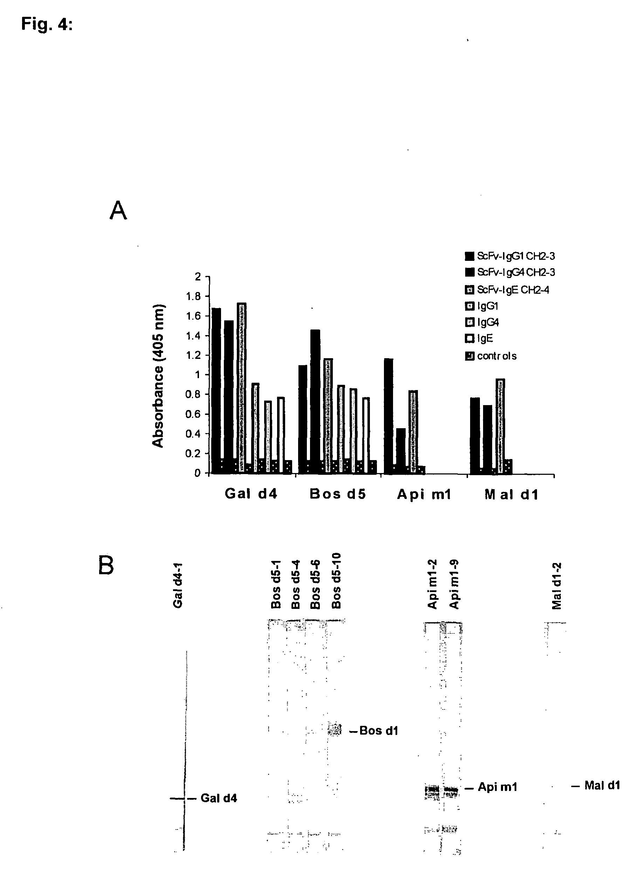

For convenient expression of scFv-derived homodimeric antibody constructs a set of modular cassettes containing leader sequences, constant immunoglobulin regions, and restriction sites for the incorporation of scFv was generated (see Figure 4).

Transfer of the anti-PLA2 scFv into the scFv-C_{H}2-3 and into the scFv-C_{H}1-3 format (IgG1,IgG4 and IgE constructs) was performed by introduction of a BsiW I site at the N-terminus (gatccgtacgtgtgggcacgtgcagctggtgc, SEQ ID NO: 13) and an Asc I site at the C-terminus (gatcggcgcgccacctaggacggtcagcttg, SEQ ID NO: 14) of the DNA encoding the variable antibody fragment by PCR. Subsequently, the DNA was ligated into the vectors containing the signal sequence and the particular constant regions.

Transfer of the anti-phl p5b scFv into the scFv-C_{H(}2-3 and into the scFv-C_{H(}1-3 format was performed by introduction of a BsiW I site at the N-terminus (gatccgtacgtgtgggcaggtgcagctgttgc, SEQ ID NO: 15) and an Asc I site at the C-terminus (gatcggcgcgccacctaggacggtcagcttg, SEQ ID NO:16) of the DNA encoding the variable antibody fragment by PCR. Subsequently, the DNA was ligated into the vectors containing the signal sequence and the particular constant regions.

Transfer of the anti-hen egg lysozyme scFv into the scFv-C_{H}2-3 and into the scFv-C_{H}1-3 format (IgG1,IgG4 and IgE constructs) was performed by introduction of a BsiW I site at the N-terminus (gatccgtacgtgtggggacgtgcagcttcaggag, SEQ ID NO: 17) and an Asc I site at the C-terminus (gatcggcgcgccttttatttccagcttggtc, SEQ ID NO: 18) of the DNA encoding the variable antibody fragment by PCR. Subsequently, the DNA was ligated into the vectors containing the signal sequence and the particular constant regions.

### EXAMPLE 5: AMPLIFICATION AND CLONING OF HETEROTETRAMERIC ANTIBODY CONSTRUCTS

### Amplification and cloning of the human κ light chain leader sequence

The signal sequence of a human κ light chain was built by amplification together with the anti-PLA2 variable light chain using overlapping primers containing an Sbf I site (ctggggctcctgctactctggctccgaggtgccagatgtcaatctgccctgactcagcctgc, SEQ ID NO: 19) and a Sal I site (gatcgtcgacatggacatgagggtccccgctcagctcctggggctcctgctactc, SEQ ID NO: 20) and a primer containing a Sgf I site (gatcgcgatcgcacctaggacggtcagcttg, SEQ ID NO: 21).

### Amplification and cloning of the human heavy chain leader sequence

The signal sequence of a human immunoglobulin heavy chain was amplified by amplification together with the anti-PLA2 variable heavy chain using overlapping primers containing an Swa I site (tgggttttccttgttgctatatttaaatgtgtccagtgtcaggtgcagctggggcag, SEQ ID NO: 22) and a Not I site (agaatgcggccgcatggaattggggctgagctgggttttccttgttgc, SEQ ID NO: 23) and a primer containing an Asc I site (gatcggcgcgccgacggtgaccagggtacc, SEQ ID NO:24).

### Amplification and cloning of human constant regions for heterotetrameric antibodies

For amplification of the γ1 constant heavy chain, a primer was used containing a Sfi I site (gatcggcccagccggcctcatttacccggagacaggga, SEQ ID NO: 25) and another primer containing an Asc I site (gatcggcgcgccagcctccaccaagggc, SEQ ID NO: 26).

For amplification of the γ4 constant heavy chain, a primer was used containing a Sfi I site (gatcggcccagccggcctttacccagagacaggga, SEQ ID NO: 27) and another primer containing an Asc I site (gatcggcgcgccagcctccaccaagggcccat, SEQ ID NO: 28).

For amplification of the ε constant heavy chain, a primer was used containing a 4xHis overhang and a Sfi I site (gatcggcccagccggcctcaatggtggtgatgtttaccgggatttacagacaccg, SEQ ID NO: 29) and another primer containing an Asc I site (gatcggcgcgccatccgtcttccccttga, SEQ ID NO: 30).

For amplification of the α constant heavy chain, a primer was used containing a 4xHis overhang and a Sfi I site (gatcggcccagccggcctcaatggtggtgatggtggacggcacctgctac, SEQ ID NO: 47) and another primer containing an Asc I site (gatcggcgcgccagcatccccgaccagcc, SEQ ID NO: 48).

### Amplification and cloning of the human κ light chain sequence

The human κ light chain constant domain was amplified from a cDNA library derived from human peripheral mononuclear cells, using one PCR primer containing a Xba I site (gatctctagactaacactctcccctgttgaagc, SEQ ID NO: 31) and another primer containing an Sgf I site (gatcgcgatcgcacgaactgtggctgcaccatctgtc, SEQ ID NO: 32). After hybridisation of the human variable and constant κ light chain the light chain sequence was introduced via the Xba I and the Sal I sites into the mammalian expression vector pBudCE4.1 (Invitrogen life technologies, Karlsruhe, Germany).

### Amplification and cloning of complete heavy and light chains

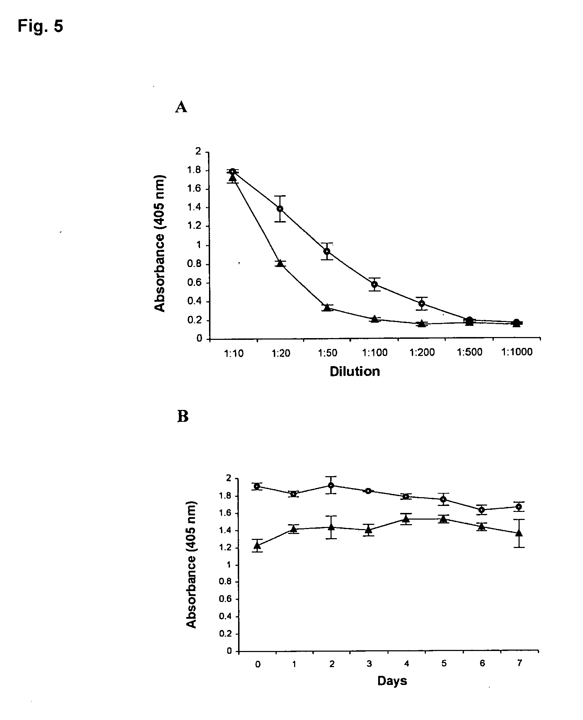

For convenient expression of heterotetrameric antibody constructs, a set of modular cassettes shown in Figure 5 was generated. Expression of the heterotetrameric IgG was achieved by inserting the IgG heavy chain and the κ light chain domains simultaneously into the mammalian expression vector pBudCE4.1 (Invitrogen life technologies, Karlsruhe, Germany). The human light and heavy chains were introduced via the Sgf I and the Sbf I sites, and the Asc I and Swa I sites, respectively. Coexpression of heavy and light chains is driven by strong CMV and EF-1α promoters.

Transfer of the anti-phl p5b vH (variable heavy) region into the heterotetrameric IgG (isotypes 1 and 4) and IgE was performed by introduction of a Swa I site at the N-terminus (gatcatatttaaatgtgtccagtgtcaggtgcagctgttgcag, SEQ ID NO: 33) and an Asc I site at the C-terminus (gatcggcgcgccgacggtgaccacggtacc, SEQ ID NO: 34) of the DNA encoding the variable antibody fragment by PCR. Transfer of the anti-phl p5b vL (variable light) region into the heterotetrameric antibodies was performed by introduction of a Sbf I site at the N-terminus (gatccctgcagggtgccagatgtcagtctgtactg, SEQ ID NO: 35) and an Sgf I site at the C-terminus (gatcgcgatcgcacctaggacggtcagctt, SEQ ID NO: 36) of the DNA encoding the variable antibody fragment by PCR.

Transfer of the anti-hen egg lysozyme vH region into the heterotetrameric IgG (isotypes 1 and 4) and IgE was performed by introduction of a Swa I site at the N-terminus (gatcatttaaatgtgtccagtgtgacgtgcagctgcag, SEQ ID NO: 37) and an Asc I site at the C-terminus (gatcggcgcgccgacggtgaccgtggtacc, SEQ ID NO: 38) of the DNA encoding the variable antibody fragment by PCR. Transfer of the anti-hen egg lysozyme vL region into the heterotetrameric antibodies was performed by introduction of a Sbf I site at the N-terminus (gatccctgcagggtgccagatgtgacatcgtgctgacc, SEQ ID NO: 39) and an Sgf I site at the C-terminus (gatcgcgatcgctttgatttccttcag, SEQ ID NO: 40) of the DNA encoding the variable antibody fragment by PCR.

### EXAMPLE 6: EXPRESSION OF ANTIBODY CONSTRUCTS

HEK-293 cells (ATCC number CRL-1573) were cultivated in DMEM (Dulbecco's modified Eagle Medium) supplemented with 10% (v/v) heat-inactivated fetal calf serum, 100 IU/ml penicillin, and 100 µg/ml streptomycin. Tissue culture reagents were obtained from Invitrogen life technologies (Karlsruhe, Germany). HEK-293 cells growing in DMEM supplemented with 10% (v/v) fetal calf serum were transfected with 2 µg of the particular expression vector using PEI (polyethylenimine, Sigma, Taufkirchen, Germany). Stable transfectants then were selected in DMEM supplemented with 10% (v/v) fetal calf serum and 100 µg/ml of zeocin (Invitrogen life technologies, Karlsruhe, Germany).

For protein expression, transfected cells were grown for 3 days as an adhesion culture. The immunoglobulins secreted by transfected HEK-293 cells were purified from the culture medium by affinity chromatography using protein A-agarose (Santa Cruz Biotechnologies, Santa Cruz, Ca.) or Ni-NTA-agarose (Qiagen, Hilden, Germany) according to the manufacturers recommendations. Examples of purified antibody constructs analyzed by SDS polyacrylamide gel electrophoresis are shown in Figure 5.

### EXAMPLE 7: PRODUCTION OF COMPOSITIONS OF HUMAN ALLERGEN-SPECIFIC IgE AND IgG4 AND IgA ANTIBODIES

For the production of reference sera with defined levels of human allergen-specific IgE antibody, 50 µl of purified recombinant antibodies diluted with PBS-fetal calf serum 1% (Biochrom AG, Berlin) were applied to microtiter plates supplied with specific allergen discs (Allergopharma, Reinbek, Germany). As a reference, 50 µl of conventional standardized sera (EAST-classes 0-5) provided by Allergopharma, were applied to paper discs coated with anti-human IgE antibodies.

After incubation of the microtiter plates at 37 °C for 60 min, the wells were rinsed 4 times with PBS-Tween (0.1 % v/v, TPBS). Subsequently, the paper discs were further incubated with 50 µl each of anti-human IgE conjugate (Allergopharma, Reinbek) and incubated at 37°C for 90 minutes. The wells were rinsed 4 times with PBS-Tween (0.1 % v/v, TPBS) and bound antibodies were visualized by the addition of 200 µl each of a p-nitrophenylphosphate solution (Allergopharma, Reinbek, Germany). After incubation of the microtiter plates at 37 °C for 60 minutes the reaction was stopped by addition of 100 µl each of 1 M NaOH. Absorbance was determined at 405 nm and 620 as reference wavelength.

### EXAMPLE 8: EPITOPE-SPECIFIC REACTIVITY OF ANTIBODY CONSTRUCTS

For assessment of epitope-specific immunoreactivity antigens were labeled with I-125 using IODO-Beads (Pierce). Just before use, the beads were washed with 500 µl of PBS, pH 7.2 per bead and dried on filter paper. One bead was added to a solution of carrier-free NaI-125 in NaOH (approximately 1 mCi per 100 µg of protein) diluted with PBS and allowed to react for 5 minutes. 100 µg of protein (hen egg lysozyme) were dissolved in PBS, added to the reaction vessel and incubated for 2-15 minutes at room temperature. The reaction was stopped by removing the solution from the reaction vessel containing IODO-Beads. Excess Na¹²⁵I or unincorporated I-125 was removed from the iodinated protein after centrifugation at 14 000 x g for 15 min at 4°C following three wash steps with PBS using Microcon YM-10-centrifugal filter unites (Millipore, Germany).

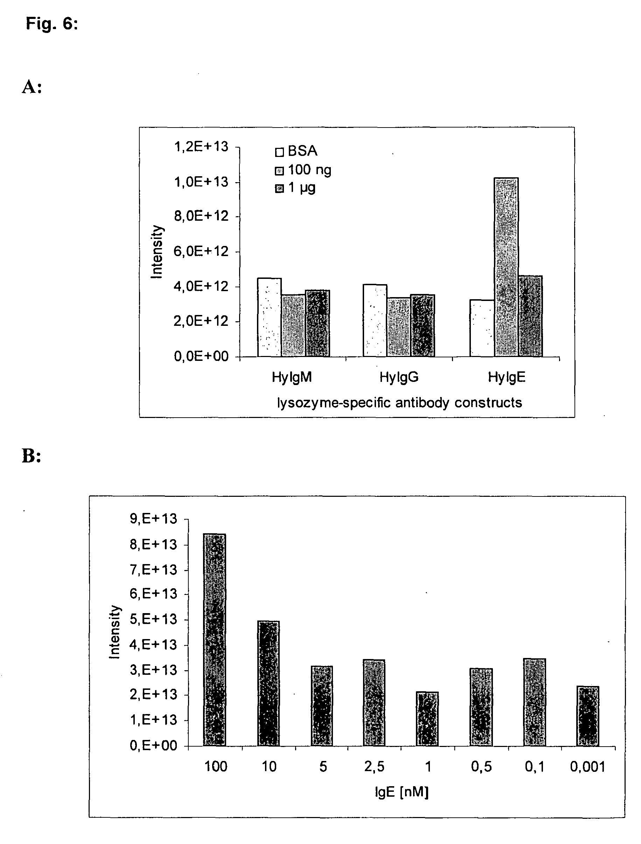

Cyanogen-bromide activated paper discs (Allergopharma, Reinbek, Germany) were applied to microtiter plates and incubated with purified recombinant chimera of IgE receptor α-chain and avian constant immunoglobulin domain Cυ1-4 protein at a concentration of 100 µg/ml in PBS at room temperature for 2 h. Paper discs were blocked with BSA (1% v/v, PBS) at room temperature for 2 h and the wells were rinsed 3 times each with PBS-Tween (0.1% v/v, TPBS) and PBS. Subsequently IgE antibody constructs diluted in BSA (1% v/v, PBS) were added and further incubated for 60 min at room temperature on a rocker platform. The wells were rinsed again 3 times each with 0.1% (v/v) TPBS and PBS and bound antibodies were visualized by the addition of I-125 labelled antigen. For assessment of epitope specificity of recombinant antibody constructs, sera from allergic patients were added in order to compete with antigen binding. After incubation for 60 min at room temperature the wells were rinsed again 3 times each with 0.1% (v/v) TPBS and PBS. Then the paper discs were removed from the wells and measurement of γ-radiation was performed using an ERASER K Screen and Personal FX PhosphoImager (BioRad, Munich, Germany) (see also Figure 6).

### TABLE LEGENDS

### Table 1: Calculation of the average number of epitopes on allergens.

Numbers are derived from calculation of the solvent accessible surface calculation using the **POPS** (**P**arameter **OP**timised **S**urfaces) server. The method is described in: Parameter optimized surfaces (POPS): analysis of key interactions and conformational changes in the ribosome. Fratemali, F. and Cavallo, L. (2002) Nucleic Acids Research 30, 2950-2960. (http://ibivu.cs.vu.nl/programs/popswwwn

As input files the available structural data of the protein data bank (PDB) are used. The list of allergens has been compiled using the SDAP (Structural Database of Allergenic Proteins) database (University of Texas Medical Branch, http://fermi.utmb.edu/SDAP/index.html)

### Table 2: Calculation of estimated numbers of potential IgE epitopes on selected allergens

Calculation is based on known IgE binding sites referenced in the SDAP database (see Tab1 and references below).
**(Alt a 1)** V. P. Kurup, H. M. Vijay, V. Kumar, L. Castillo, and N. Elms, IgE binding synthetic peptides of Alt a 1, a major allergen of Alternaria alternata, Peptides 2003, 00, 00-00.
**(Ara h 1)** D. S. Shin, C. M. Compadre, S. J. Maleki, R. A. Kopper, H. Sampson, S. K. Huang, A. W. Burks, and G. A. Bannon, Biochemical and structural analysis of the IgE binding sites on Ara h 1, an abundant and highly allergenic peanut protein, J. Biol. Chem. 1998, 273, 13753-13759.
**(Ara h 2)** J. S. Stanley, N. King, A. W. Burks, S. K. Huang, H. Sampson, G. Cockrell, R. M. Helm, C. M. West, and G. A. Bannon, Identification and mutational analysis of the immunodominant IgE binding epitopes of the major peanut allergen Ara h 2, Arch. Biochem. Biophys. 1997, 342, 244-253.
**(Asp f 1)** V. P. Kurup, B. Banerjee, P. S. Murali, P. A. Greenberger, M. Krishnan, V. Hari, and J. N. Fink, Immunodominant peptide epitopes of allergen, Asp f 1 from the fungus Aspergillus fumigatus, Peptides 1998, 19, 1469-1477.
**(Asp f 2)** B. Banerjee, P. A. Greenberger, J. N. Fink, and V. P. Kurup, Conformational and linear B-cell epitopes of Asp f 2, a major allergen of Aspergillus fumigatus, bind differently to immunoglobulin E antibody in the sera of allergic bronchopulmonary aspergillosis patients, Infect. Immun. 1999, 67, 2284-2291.
**(Asp f 3)** H. Ramachandran, V. Jayaraman, B. Banerjee, P. A. Greenberger, K. J. Kelly, J. N. Fink, and V. P. Kurup, IgE binding conformational epitopes of Asp f 3, a major allergen of Aspergillus fumigatus, Clin. Immun. 2002, 103, 324-333.
**(Asp f 13)** L.-P. Chow, S.-L. Liu, C.-J. Yu, H.-K. Liao, J.-J. Tsai, and T.-K. Tang, Identification and expression of an allergen Asp f 13 from Aspergillus fumigatus and epitope mapping using human IgE antibodies and rabbit polyclonal antibodies, Biochem. J. 2000, 346, 423-431.
**(Bet v 2)** A. A. Fedorov, T. Ball, N.M. Mahoney, R. Valenta and S.C. Almo, The molecular basis for allergen cross-reactivity: crystal structure and IgE-epitope mapping of birch pollen profiling, Structure 1997, 5(1) , 33-45.
**(Bos d 5)** K.-M. Järvinen, P. Chatchatee, L. Bardina, K. Beyer and H.A. Sampson, IgE and IgG binding epitopes on a-Lactalbumin and b-Lactoglobulin in cow's milk allergy, Int. Arch. Immunol. 2001, 126, 111-118.
**(Cry j 2)** Y. Tamura, J. Kawaguchi, N. Serizawa, K. Hirahara, A. Shiraishi, H. Nigi, Y. Taniguchi, M. Toda, S. Inouye, T. Takemori, and M. Sakaguchi, Analysis of sequential immunoglobulin E-binding epitope of Japanese cedar pollen allergen (Cry j 2) in humans, monkeys and mice, Clin. Exp. Allergy 2003, 33, 211-217.
**(Gal d 1)** Y. Mine and J. W. Zhang, Identification and fine mapping of IgG and IgE epitopes in ovomucoid, Biochem. Biophys. Res. Commun. 2002, 292, 1070-1074.
**(Hev b 1 / Hev b 3)** B. Banerjee, K. Kanitpong, J. N. Fink, M. Zussman, G. L. Sussman, K. J. Kelly, and V. P. Kurup, Unique and shared IgE epitopes of Hev b 1 and Hev b 3 in latex allergy, Mol. Immunol. 2000, 37, 789-798.
**(Hev b 5)** D. H. Beezhold, V. L. Hickey, and G. L. Sussman, Mutational analysis of the IgE epitopes in the latex allergen Hev b 5, J. Allergy Clin. Immunol. 2001, 107, 1069-1076.
**(Jun a 1)** T. Midoro-Horiuti, V. Mathura, C. H. Schein, W. Braun, S. Yu, M. Watanabe, J. C. Lee, E. G. Brooks, and R. M. Goldblum, Major linear IgE epitopes of mountain cedar pollen allergen Jun a 1 map to the pectate lyase catalytic site, Mol. Immunol. 2003, 40, 555-562.
**(Jun a 3)** K. V. Soman, T. Midoro-Horiuti, J. C. Ferreon, R. M. Goldblum, E. G. Brooks, A. Kurosky, W. Braun, and C. H. Schein, Homology modeling and characterization of IgE binding epitopes of mountain cedar allergen Jun a 3, Biophys. J. 2000, 79, 1601-1609.
**(Par j 1 / Par j 2)** J. A. Asturias, N. Gomez-Bayon, J. L. Eseverri, and A. Martinez, Par j 1 and Par j 2, the major allergens from Parietaria judaica pollen, have similar immunoglobulin E epitopes, Clin. Exp. Allergy 2003, 33, 518-524.
**(Pen n 18)** C.-J. Yu, Y.-M. Chen, S.-N. Su, F. Forouhar, S.-H. Lee, and L.-P. Chow, Molecular and immunological characterization and IgE epitope mapping of Pen n 18, a major allergen of Penicillium notatum, Biochem. J. 2002, 363, 707-715.

### FIGURE LEGENDS

**Fig. 1****: Schematic representation of the immunoglobulins**
   A: In the IgG molecule, the scFv-CH1-3 and the scFv-CH2-3, the constant regions of the heavy and light chains are presented in grey, the variable regions in light grey, the linker region joining the variable regions in the scFv-CH1-3 and the scFv-CH2-3 in grey.
   B+C: Modular expression cassettes for expression of immunoglobulins. Shown are the expression cassettes for pcDNA3.1-zeo (B) and pBudCE4.1 (C). Restriction sites for cloning of variable and constant regions of the cDNA are indicated.
**Fig. 2****: Western blot analysis of recombinant IgG and IgE proteins**
   Purified scFv-C_{H}2-C_{H}3 proteins (lanes 1,2, and 5) and full Ig proteins (lanes 3, 4, and 7) were separated by 7.5 % SDS-PAGE under non-reducing conditions and transferred to a PVDF-membrane and incubated with the particular anti-human IgG-AP conjugate or anti-human IgE-AP conjugate (diluted 1: 10.000 or 1:1.000 in MPBS). Bound antibodies were visualised using NBT/BCIP as substrate.
**Fig. 3****: Immunoreactivity of the selected phage antibodies**
   The immunoreactivity of selected clones against the particular allergens (filled bars, controls by omission of antigen: open bars) was analysed by ELISA as described in the Examples.
**Fig. 4****: Immunoreactivity of the recombinant antibodies**
   A: Immunoreactivity of different recombinant antibody constructs produced on the basis of different scFv was assessed by ELISA. Bound constructs were visualised using monoclonal anti-human IgG1, IgG4, and IgE alkaline phosphatase conjugate.
   B: Recombinant scFv-based IgE antibodies were employed in Western blot based allergen detection by applying antibodies to the particular AlaBLOT strips (F1, F2, I1 and F49) and detection was performed using monoclonal anti-human IgE AP conjugate according to the manufacturer's recommendations.
**Fig. 5****: Stability of the recombinant antibodies in human serum**
   A: Reactivities of recombinant scFv-based IgE antibodies Bos d5-1 and Bos d5-4 diluted in human serum of a non-allergic individual against the allergen were assessed by ELISA. Bound IgE constructs were visualised using monoclonal anti-human IgE AP conjugate.
   B: Long-term stability of recombinant scFv-based IgE antibodies Bos d5-1 and Bos d54 in human serum was assessed by ELISA. Bound IgE constructs were visualised as above.
**Fig. 6****: Epitope-specific reactivity of monoclonal antibody constructs**
   Purified recombinant chimera of IgE receptor α-chain and avian constant immunoglobulin domain Cυ1-4 protein was coupled to cyanogen-bromide-activated paper discs as described above. A: Subsequently monoclonal lysozyme-specific antibody constructs of IgM, IgG1 and IgE isotype were diluted in BSA (1% v/v, PBS) and applied to the wells. **B:** Concentration series of IgE. Bound antibodies were visualized by the addition of I-125 labelled antigen at a concentration of 1 µg/ml diluted in BSA (1% v/v, PBS). Measurement of γ-radiation was performed using an ERASER K Screen and Personal FX PhosphoImager (BioRad, Munich, Germany).

**Table 1**

| Antigen | Protein | Organism | Common | PDB code | Size | Surface (Å²) | Surface epitopes | Surface epitopes / kDa |
|---|---|---|---|---|---|---|---|---|
| Amb t 5 | Ra5G | Ambrosia trifida | Ragweed | 1BBG | 4.3 kDa | 2438.3 | 2.57 | 0.6 |
| Api m 1 | Phospholipase A2 | Apis mellifera | Bee | IPOC | 16-20 kDa | 7606.4 | 8.0 | 0.4 |
| Api m 2 | Hyaluronidase | Apis mellifera | Bee | 1FCQ | 43 kDa | 15905.5 | 16.74 | 0.39 |
| Api m 4 | Melittin | Apis mellifera | Bee | 2MLT | 3 kDa | 3885.7 | 4.09 | 1.36 |
| Ara t 8 | Profilin | Ara. thaliana | Cress | 1A0K | 14.2 kDa | 7080.1 | 7.45 | 0.52 |
| Asp f 1 | Mitogillin | Asp. fumigatus | Fungi | 1AQZ | 16.8 kDa | 16037.6 | 16.88 | 1.0 |
| Asp f 6 | Dismutase | Asp. fumigatus | Fungi | 1KKC | 23.3 kDa | 8793.2 | 9.26 | 0.4 |
| Bet v 1 | PR10 | Betulla verrucosa | Birch | 1BV1 | 17.4 kDa | 5215.3 | 5.49 | 0.32 |
| Bet v 2 | Profilin | Betulla verrucosa | Birch | 1CQA | 14.3 kDa | 6493.9 | 6.84 | 0.48 |
| Bos d 4 | a-Lactoglobulin | Bos domesticus | Cow | 1HFZ | 14.2 kDa | 7246.9 | 7.63 | 0.54 |
| Bos d 5 | b-Lactoglobulin | Bos domesticus | Cow | 1B8E | 18.2 kDa | 9546.5 | 10.05 | 0.55 |
| Bos d 5 | b-Lactoglobulin | Bos domesticus | Cow | 1QG5 | 18.2 kDa | 9618.4 | 10.12 | 0.56 |
| Der f2 | - | Der. farinae | Mite | 1AHK | 15.8 kDa | 7785.2 | 8.19 | 0.52 |
| Der p2 | - | Der. pteronyssinus | Mite | 1KTJ | 16 kDa | 7588.8 | 7.99 | 0.5 |
| Equ c 1 | Lipocalin | Equus caballus | Horse | 1EW3 | 20 kDa | 8907.4 | 9.38 | 0.47 |
| Gal d 3 | Ovotransferrin | Gallus domesticus | Chicken | 1NNT | 75.8 kDa | 15952.9 | 16.79 | 0.22 |
| Gal d 4 | Lysozyme | Gallus domesticus | Chicken | 2LYZ | 16.2 kDa | 6951.3 | 7.32 | 0.45 |
| Hev b 8 | Profilin | Hevea brasiliensis | Latex | 1G5U | 14 kDa | 11982 | 12.61 | 0.9 |
| Mus m 1 | MUP | Mus musculus | Mouse | 1MUP | 18.7 kDa | 8943.5 | 9.41 | 0.5 |
| Phl p 1 | - | Phleum pratense | Timothy | 1N10 | 26.1 kDa | 12145.6 | 12.78 | 0.49 |
| Phl p 2 | - | Phleum pratense | Timothy | 1WHO | 10.8 kDa | 6099.5 | 6.42 | 0.59 |
| Phl p 6 | - | Phleum pratense | Timothy | 1NLX | 11.8 kDa | 5429.5 | 5.72 | 0.48 |
| Pru av 1 | - | Prunus avium | Cherry | 1E09 | 17.7 kDa | 9742.8 | 10.26 | 0.58 |
| Ves v 5 | Antigen 5 | Vespula vulgaris | Wasp | 1QNX | 25.8 kDa | 11657.1 | 12.27 | 0.47 |
| Zea m14 | - | Zea mays | Corn | 1MZM | 11.7 kDa | 5099.5 | 5.37 | 0.46 |

| | | | | | | | | |
|---|---|---|---|---|---|---|---|---|
| *Estimated antibody epitope area: 950 Å | | | | | | | | |

**Table 2**

| Antigen | Size | Identified IgE binding peptides | Identified IgE binding peptides / kDa | Estimated IgE epitopes / kDa |
|---|---|---|---|---|
| Alt a 1 | 15.2 kDa | 2 | 0.13 | 0.043 |
| Ara h 1 | 67.7 kDa | 21 | 0.31 | 0.103 |
| Ara h 2 | 17.5 kDa | 10 | 0.57 | 0.190 |
| Asp f 1 | 16.8 kDa | 13 | 0.77 | 0.257 |
| Asp f 2 | 31.2 kDa | 9 | 0.29 | 0.096 |
| Asp f 3 | 18.4 kDa | 7 | 0.38 | 0.127 |
| Asp f 13 | 28.7 kDa | 5 | 0.17 | 0.057 |
| Bet v 2 | 14.3 kDa | 3 | 0.21 | 0.070 |
| Bos d 5 | 18.2 kDa | 7 | 0.38 | 0.127 |
| Bos d 5 | 18.2 kDa | 7 | 0.38 | 0.127 |
| Cry j 2 | 42.2 kDa | 4 | 0.09 | 0.030 |
| Gal d 1 | 20.1 kDa | 9 | 0.45 | 0.150 |
| Hev b 1 | 14.6 kDa | 8 | 0.55 | 0.183 |
| Hev b 3 | 22.3 kDa | 11 | 0.49 | 0.163 |
| Hev b 5 | 15.9 kDa | 11 | 0.69 | 0.230 |
| Jun a 1 | 37.6 kDa | 4 | 0.11 | 0.037 |
| Jun a 3 | 21 kDa | 5 | 0.24 | 0.080 |
| Par j 1 | 15 kDa | 5 | 0.33 | 0.110 |
| Par j 2 | 11.3 kDa | 8 | 0.71 | 0.237 |
| Pen n 18 | 52.4 kDa | 9 | 0.17 | 0.057 |
| | | | | |
| Average value | | | | 0.124 +/- 0.067 |

| | | | | |
|---|---|---|---|---|
| *assuming 3 linear IgE binding peptides are shared by an IgE epitope. | | | | |

## Claims

1. Method for generating a composition comprising antibodies capable of binding to all epitopes on an allergen X recognized by antibodies of one or more specific isotype(s), comprising steps of
a) generating a plurality of allergen-specific antibodies capable of binding to the allergen X,
b) combining a plurality of the generated antibodies and testing whether essentially complete inhibition of binding of the allergen X to human IgE, IgG4 or IgA antibodies in a pool serum of patients allergic to said allergen or obtained from said serum is achieved,
c) in case essentially complete inhibition is not achieved in step b), repeating steps a) and b);
d) in case essentially complete inhibition is achieved, reducing the number of antibodies to the minimal number of antibodies sufficient for essentially complete inhibition by a method wherein
i) groups of the antibodies obtained in step a) are generated, comprising different numbers and combinations of antibodies;
ii) said groups are tested for essentially complete inhibition of binding of the allergen X to IgE, IgG4 and/or IgA antibodies in a pool serum of patients allergic to said allergen or obtained from said sera;
iii) wherein, in case one or more group effects essentially complete inhibition in step ii), steps i) and ii) are repeated with subcombinations of the antibodies from said group or groups until the minimal number of antibodies in said group or groups is identified which effects essentially complete inhibition;
iv) wherein, in case essentially complete inhibition is not achieved in step ii) or iii), steps i), ii) and iii) are repeated with different groups of antibodies;
and wherein the group identified in step d), iii) is said composition.

2. Method of claim 1, wherein the composition comprises antibodies capable of binding to all epitopes recognized by IgE, IgG4 and/or IgA antibodies.

3. Method of any of the preceding claims, wherein the composition comprises antibodies capable of binding to all epitopes recognized by human IgE, IgG4 and/or IgA antibodies.

4. Method of any of the preceding claims, wherein the composition comprises recombinant antibodies.

5. Method of any of the preceding claims, wherein the composition comprises scFv or Fab antibodies.

6. Method of any of the preceding claims, wherein in step a, the plurality of antibodies is generated by selecting antibodies from a natural, semisynthetic or synthetic library of antibodies.

7. Method of any of the preceding claims, wherein in step b, the plurality of antibodies is 2 to 200 antibodies.

8. Method of any of the preceding claims, wherein the composition comprises 0,06-0,19 antibodies / 1kDa of the allergen X.

9. Composition comprising a plurality of recombinant antibodies, wherein the antibodies in the composition are capable of binding to all epitopes of an allergen X recognized by antibodies of one or more specific isotype(s), obtainable by the method of claims 1 to 8.

10. Method of identifying all epitopes on an allergen X recognized by antibodies of one or more specific isotype(s), comprising carrying out the method of claims 1 to 8 and identifying the epitopes recognized by the antibodies in the obtained composition.

11. Method of generating a hypoallergenic molecule for specific immunotherapy comprising carrying out the method of claim 10, wherein the specific isotype is IgE, and modifying amino acid residues of the epitopes identified thereby to reduce recognition of the hypoallergenic molecule by the IgE antibodies.

12. Method of claim 11, further comprising carrying out the method of claim 10, wherein the specific isotype is selected from the group of IgG4 and IgA, and maintaining amino acids of the epitopes identified thereby to maintain recognition of the hypoallergenic molecule by the IgG4 and/or IgA antibodies.

13. Method of claims 11 or 12, wherein the allergenicity of the hypoallergic molecule is reduced to 20% or less while at least 50% of IgA and/or IgG4 epitopes are maintained.

14. Hypoallergenic molecule obtainable by the method of claims 11 to 13.


SEQUENCE LISTING

<110>  University of Hamburg

<120>  Antibody compositions specific for IgE, IgG4 and IgA epitopes as
       tools for the design of hypoallergenic molecules for specific
       immunotherapy

<130>  P 66303

<160>  51

<170>  PatentIn version 3.4

<210>  1
<211>  44
<212>  DNA
<213>  Artificial

<220>
<223>  Primer

<400>  1
gatcggtacc gatcggcgcg cccaaatctt gtgacaaaac tcac                        44


<210>  2
<211>  41
<212>  DNA
<213>  Artificial

<220>
<223>  Primer

<400>  2
gatctctaga tcatttaccc ggagacaggg agaggctctt c                           41


<210>  3
<211>  44
<212>  DNA
<213>  Artificial

<220>
<223>  Primer

<400>  3
gatcggtacc gatcggcgcg cccaaatctt gtgacaaaac tcac                        44


<210>  4
<211>  32
<212>  DNA
<213>  Artificial

<220>
<223>  Primer

<400>  4
gatcggcgcg ccagcctcca ccaagggccc at                                     32


<210>  5

<211>  31
<212>  DNA
<213>  Artificial

<220>
<223>  Primer

<400>  5
agtatctaga tcatttaccc agagacaggg a                                    31


<210>  6
<211>  32
<212>  DNA
<213>  Artificial

<220>
<223>  Primer

<400>  6
gatcggcgcg cccagcaaca ccaaggtgga ca                                   32


<210>  7
<211>  31
<212>  DNA
<213>  Artificial

<220>
<223>  Primer

<400>  7
agtatctaga tcatttaccc agagacaggg a                                    31


<210>  8
<211>  42
<212>  DNA
<213>  Artificial

<220>
<223>  Primer

<400>  8
gatcggcgcg ccttccacca agggcccatc cgtcttcccc ct                        42


<210>  9
<211>  48
<212>  DNA
<213>  Artificial

<220>
<223>  Primer

<400>  9
gatctctaga tcaatggtgg tgatgtttac cgggatttac agacaccg                  48


<210>  10
<211>  29
<212>  DNA
<213>  Artificial

<220>
<223>  Primer

<400>  10
gatcggcgcg cccaccgtga agatcttac                                          29


<210>  11
<211>  41
<212>  DNA
<213>  Artificial

<220>
<223>  Primer

<400>  11
gtacaagctt gctagcaaga tggaatcaca gacccaggtc c                            41


<210>  12
<211>  40
<212>  DNA
<213>  Artificial

<220>
<223>  Primer

<400>  12
gtacacgcgt tgtaaggact caccccacag gtaccagaaa                              40


<210>  13
<211>  32
<212>  DNA
<213>  Artificial

<220>
<223>  Primer

<400>  13
gatccgtacg tgtgggcacg tgcagctggt gc                                      32


<210>  14
<211>  31
<212>  DNA
<213>  Artificial

<220>
<223>  Primer

<400>  14
gatcggcgcg ccacctagga cggtcagctt g                                       31


<210>  15
<211>  32
<212>  DNA
<213>  Artificial

<220>
<223>  Primer

<400> 15
gatccgtacg tgtgggcagg tgcagctgtt gc 32

<210> 16
<211> 31
<212> DNA
<213> Artificial

<220>
<223> Primer

<400> 16
gatcggcgcg ccacctagga cggtcagctt g 31

<210> 17
<211> 34
<212> DNA
<213> Artificial

<220>
<223> Primer

<400> 17
gatccgtacg tgtggggacg tgcagcttca ggag 34

<210> 18
<211> 31
<212> DNA
<213> Artificial

<220>
<223> Primer

<400> 18
   gatcggcgcg ccttttattt ccagcttggt c 31

<210> 19
<211> 62
<212> DNA
<213> Artificial

<220>
<223> Primer

<400> 19
ctggggctcc tgctactctg gctccgaggt gccagatgtc aatctgccct gactcagcct 60

gc 62

<210> 20
<211> 55
<212> DNA
<213> Artificial

<220>
<223> Primer

<400>  20
gatcgtcgac atggacatga gggtccccgc tcagctcctg gggctcctgc tactc          55

<210>  21
<211>  31
<212>  DNA
<213>  Artificial

<220>
<223>  Primer

<400>  21
gatcgcgatc gcacctagga cggtcagctt g                                    31

<210>  22
<211>  57
<212>  DNA
<213>  Artificial

<220>
<223>  Primer

<400>  22
tgggttttcc ttgttgctat atttaaatgt gtccagtgtc aggtgcagct ggggcag        57

<210>  23
<211>  48
<212>  DNA
<213>  Artificial

<220>
<223>  Primer

<400>  23
agaatgcggc cgcatggaat tggggctgag ctgggttttc cttgttgc                  48

<210>  24
<211>  30
<212>  DNA
<213>  Artificial

<220>
<223>  Primer

<400>  24
gatcggcgcg ccgacggtga ccagggtacc                                      30

<210>  25
<211>  38
<212>  DNA
<213>  Artificial

<220>
<223>  Primer

<400>  25
gatcggccca gccggcctca tttacccgga gacaggga                             38

<210> 26
<211> 28
<212> DNA
<213> Artificial

<220>
<223> Primer

<400> 26
gatcggcgcg ccagcctcca ccaagggc 28

<210> 27
<211> 35
<212> DNA
<213> Artificial

<220>
<223> Primer

<400> 27
gatcggccca gccggccttt acccagagac aggga 35

<210> 28
<211> 32
<212> DNA
<213> Artificial

<220>
<223> Primer

<400> 28
gatcggcgcg ccagcctcca ccaagggccc at 32

<210> 29
<211> 55
<212> DNA
<213> Artificial

<220>
<223> Primer

<400> 29
gatcggccca gccggcctca atggtggtga tgtttaccgg gatttacaga caccg 55

<210> 30
<211> 29
<212> DNA
<213> Artificial

<220>
<223> Primer

<400> 30
gatcggcgcg ccatccgtct tccccttga 29

<210> 31
<211> 33

<212>  DNA
<213>  Artificial

<220>
<223>  Primer

<400>  31
gatctctaga ctaacactct cccctgttga agc                                    33


<210>  32
<211>  37
<212>  DNA
<213>  Artificial

<220>
<223>  Primer

<400>  32
gatcgcgatc gcacgaactg tggctgcacc atctgtc                                37


<210>  33
<211>  43
<212>  DNA
<213>  Artificial

<220>
<223>  Primer

<400>  33
gatcatattt aaatgtgtcc agtgtcaggt gcagctgttg cag                         43


<210>  34
<211>  30
<212>  DNA
<213>  Artificial

<220>
<223>  Primer

<400>  34
gatcggcgcg ccgacggtga ccacggtacc                                        30


<210>  35
<211>  35
<212>  DNA
<213>  Artificial

<220>
<223>  Primer

<400>  35
gatccctgca gggtgccaga tgtcagtctg tactg                                  35


<210>  36
<211>  30
<212>  DNA
<213>  Artificial

<220>
<223>  Primer

<400>  36
gatcgcgatc gcacctagga cggtcagctt                                        30


<210>  37
<211>  38
<212>  DNA
<213>  Artificial

<220>
<223>  Primer

<400>  37
gatcatttaa atgtgtccag tgtgacgtgc agctgcag                               38


<210>  38
<211>  30
<212>  DNA
<213>  Artificial

<220>
<223>  Primer

<400>  38
gatcggcgcg ccgacggtga ccgtggtacc                                        30


<210>  39
<211>  38
<212>  DNA
<213>  Artificial

<220>
<223>  Primer

<400>  39
gatccctgca gggtgccaga tgtgacatcg tgctgacc                               38


<210>  40
<211>  27
<212>  DNA
<213>  Artificial

<220>
<223>  Primer

<400>  40
gatcgcgatc gctttgattt ccttcag                                           27


<210>  41
<211>  18
<212>  DNA
<213>  Artificial

<220>
<223>  Primer

<400>  41
gccgtgacgt tggacgag                                                    18


<210>  42
<211>  23
<212>  DNA
<213>  Artificial

<220>
<223>  Primer

<400>  42
ggaggagacg atgacttcgg tcc                                              23


<210>  43
<211>  23
<212>  DNA
<213>  Artificial

<220>
<223>  Primer

<400>  43
ctgactcagc cgtcctcggt gtc                                              23


<210>  44
<211>  23
<212>  DNA
<213>  Artificial

<220>
<223>  Primer

<400>  44
taggacggtc aggccagagc cac                                              23


<210>  45
<211>  29
<212>  DNA
<213>  Artificial

<220>
<223>  Primer

<400>  45
gatcggcgcg cccgcatccc cgaccagcc                                        29


<210>  46
<211>  43
<212>  DNA
<213>  Artificial

<220>
<223>  Primer

<400>  46
gatctctaga tcaatggtgg tgatggtgga cggcacctgc tac                        43

<210>  47
<211>  50
<212>  DNA
<213>  Artificial

<220>
<223>  Primer

<400>  47
gatcggccca gccggcctca atggtggtga tggtggacgg cacctgctac                    50


<210>  48
<211>  29
<212>  DNA
<213>  Artificial

<220>
<223>  Primer

<400>  48
gatcggcgcg ccagcatccc cgaccagcc                                           29


<210>  49
<211>  31
<212>  DNA
<213>  Artificial

<220>
<223>  Primer

<400>  49
gatcgctagc atgaagaacc atttgctttt c                                        31


<210>  50
<211>  31
<212>  DNA
<213>  Artificial

<220>
<223>  Primer

<400>  50
gatctctaga ttagtcagga tagcaggcat c                                        31

<210>  51
<211>  18
<212>  PRT
<213>  Artificial

<220>
<223>  linker

<400>  51

Gly Gly Ser Ser Arg Ser Ser Ser Ser Gly Gly Gly Gly Ser Gly Gly
1               5                   10                  15

Gly Gly